# DEMANDE DE BREVET EUROPEEN

(11) **EP 1 195 156 A1**
(43) Date de publication de la demande: **10.04.2002**
(21) Numéro de dépôt: 01402443.4
(22) Date de dépôt: 24.09.2001
(51) Int. Cl.: A61K 7/48

(54) **Utilisation d'un extrait d'Ericaceae pour traîter les signes du vieillissement cutané**

(30) Priorité: 05.10.2000 FR 0012737
(71) Demandeur: L'OREAL, 75008 Paris (FR)
(72) Inventeur: Fagot, Dominique, 75020 Paris (FR)
(74) Mandataire: Galup, Cédric Olivier Nicolas

(57) **Abrégé**

L'invention se rapporte à l'utilisation d'au moins un extrait d'au moins un végétal de la famille des Ericaceae, dans ou pour la préparation d'une composition destinée à traiter, de manière préventive et/ou curative, les signes cutanés du vieillissement. En particulier, l'extrait ou la composition sont destinés à inhiber la dégradation de la peau et/ou des muqueuses par l'inhibition des collagénases.

## Description

L'invention se rapporte à l'utilisation d'au moins un extrait d'au moins un végétal de la famille des Ericaceae, dans ou pour la préparation d'une composition destinée à traiter, de manière préventive et/ou curative, les signes cutanés du vieillissement. En particulier, l'extrait ou la composition sont destinés à inhiber la dégradation de la peau et/ou des muqueuses par l'inhibition des collagénases. L'invention a également pour objet un procédé de traitement cosmétique de la peau et/ou des muqueuses.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses. Dans une peau normale, c'est à dire non pathologique ni cicatricielle, le fibroblaste est à l'état quiescent, c'est à dire non prolifératif, peu actif d'un point de vue métabolique et non mobile.

Ce sont les fibres de collagène qui assurent la solidité du derme. Les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.
Les fibres de collagènes sont constamment renouvelées mais ce renouvellement diminue avec l'âge ce qui entraîne un amincissement du derme. Cet amincissement du derme est également dû à des causes pathologiques comme par exemple l'hypersécrétion d'hormones corticoïdes, certaines pathologies ou encore des carences vitaminiques (cas de la vitamine C dans le scorbut). Il est également admis que des facteurs extrinsèques comme les rayons ultraviolets, le tabac ou certains traitements (Glucocorticoïdes, vitamine D et dérivés par exemple) ont également un effet sur la peau et sur son taux de collagène.

Cependant, divers facteurs entraînent la dégradation du collagène, avec toutes les conséquences que l'on peut envisager sur la structure et/ou la fermeté de la peau et/ou des muqueuses.

Bien que très résistantes, les fibres de collagène sont sensibles à certaines enzymes appelées collagénases. Une dégradation des fibres de collagène entraîne l'apparence de peau molle et ridée que l'être humain, préférant l'apparence d'une peau lisse et tendue, cherche depuis toujours à combattre.

Les collagénases font partie d'une famille d'enzymes appelées métalloprotéinases (MMPs) qui sont elles-mêmes les membres d'une famille d'enzymes protéolytiques (endoprotéases) qui possèdent un atome de zinc coordonné à 3 résidus cystéine et une méthionine dans leur site actif et qui dégradent les composants macromoléculaires de la matrice extracellulaire et des lames basales à pH neutre (collagène, élastine, etc. ...). Très largement répandues dans le monde vivant, ces enzymes sont présentes, mais faiblement exprimées, dans des situations physiologiques normales comme la croissance des organes et le renouvellement des tissus.

Leur surexpression chez l'homme et leur activation sont cependant liées à de nombreux processus, parfois pathologiques, qui impliquent la destruction et le remodelage de la matrice. Cela entraîne soit une résorption non contrôlée de la matrice extracellulaire, soit inversement l'installation d'un état de fibrose.

La famille des métalloprotéinases est constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa) ), les stromélysines (MMP-3) dont le large spectre d'activité s'adresse aux protéines de la matrice extracellulaire telles que les glycoprotéines (fibronectine, laminine), les protéoglycannes, etc., ou encore les métalloprotéinases membranaires.
L'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit.

Par ailleurs à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée.

On comprend alors à la lecture de ce qui précède l'importance du collagène dans la structure des tissus, particulièrement de la peau et/ou des muqueuses, et l'importance qu'il y a à combattre sa dégradation pour ainsi lutter contre le vieillissement qu'il soit chronobiologique ou photo-induit et ses conséquences, l'amincissement du derme et/ou la dégradation des fibres de collagène ce qui entraînent l'apparence de peau molle et ridée.

Un des buts de la présente invention est donc de pouvoir disposer d'un produit nouveau qui présente un effet inhibiteur des collagénases et si possible pas d'effets secondaires notables.

Or, de manière surprenante et inattendue la demanderesse a maintenant découvert qu'un extrait d'au moins un végétal de la famille des Ericaceae présente une activité inhibitrice de l'activité des collagénases.

A la connaissance de la demanderesse l'activité inhibitrice de l'activité des collagénases d'un extrait d'au moins un végétal de la famille des Ericaceae, n'a jamais été décrite.

Ainsi, l'invention a pour objet premier l'utilisation d'au moins un extrait d'au moins un végétal de la famille des Ericaceae, dans ou pour la préparation d'une composition, l'extrait ou la composition étant destinés à traiter, de manière préventive et/ou curative, les signes cutanés du vieillissement.

Par signes cutanés du vieillissement on entend toutes modifications de l'aspect extérieur de la peau dues au vieillissement qu'il soit chronobiologique et/ou photo-induit, comme par exemple les rides et ridules, la peau flétrie, la peau mole, la peau amincie, le manque d'élasticité et/ou de tonus de la peau, mais également toutes modifications internes de la peau qui ne se traduisent pas systématiquement par un aspect extérieur modifié, comme par exemple toutes dégradations internes de la peau, particulièrement du collagène, consécutives à une exposition aux rayonnements ultra-violets.

L'invention a pour second objet l'utilisation d'au moins un extrait d'au moins un végétal de la famille des Ericaceae, dans ou pour la préparation d'une composition, l'extrait ou la composition étant destinés à inhiber l'expression des protéases de la matrice extracellulaire, particulièrement des métalloprotéinases et encore plus particulièrement de la métalloprotéinase de type 1.

L'invention a pour troisième objet l'utilisation d'au moins un extrait d'au moins un végétal de la famille des Ericaceae, dans ou pour la préparation d'une composition, l'extrait ou la composition étant destinés à traiter les atteintes cutanées liées à la ménopause.

L'invention a pour quatrième objet l'utilisation d'au moins un extrait d'au moins un végétal de la famille des Ericaceae, dans ou pour la préparation d'une composition, l'extrait ou la composition étant destinés à lutter contre les rides et ridules.

L'invention a pour cinquième objet l'utilisation d'au moins un extrait d'au moins un végétal de la famille des Ericaceae, dans ou pour la préparation d'une composition, l'extrait ou la composition étant destinés à lutter contre la peau flétrie.

L'invention a pour sixième objet l'utilisation d'au moins un extrait d'au moins un végétal de la famille des Ericaceae, dans ou pour la préparation d'une composition, l'extrait ou la composition étant destinés à lutter contre la peau molle.

L'invention a pour septième objet l'utilisation d'au moins un extrait d'au moins un végétal de la famille des Ericaceae, dans ou pour la préparation d'une composition, l'extrait ou la composition étant destinés à lutter contre la peau amincie.

L'invention a pour huitième objet l'utilisation d'au moins un extrait d'au moins un végétal de la famille des Ericaceae, dans ou pour la préparation d'une composition, l'extrait ou la composition étant destinés à lutter contre le manque d'élasticité et/ou de tonus de la peau.

La famille des Ericaceae comprend environ une centaine de genres parmi lesquels on peut citer à titre d'exemple les genres Erica, Vaccinium, Calluna, Cassiope ou encore Rhododendron.

Ainsi, l'extrait d'Ericaceae de l'invention est un extrait préparé à partir de matériel issu d'au moins un végétal appartenant à un genre choisi parmi les genres Erica, Vaccinium, Calluna, Cassiope ou encore Rhododendron.

Préférentiellement selon l'invention le végétal appartient au genre Vaccinium.

Le genre Vaccinium, comporte plus de 450 espèces parmi lesquelles on peut citer les espèces *Vaccinium myrtillus, Vaccinium angustifollium, Vaccinium arboreum, Vaccinium arctostaphylos, Vaccinium caespitosum, Vaccinium corymbosum, Vaccinium hirsutum, Vaccinium macrocarpum, Vaccinium ovatum, Vaccinium oxycoccos, Vaccinium stamineum, Vaccinium uliginosum, Vaccinium urceolatum, Vaccinium vitis-idaea*.

Ainsi, l'extrait de végétal du genre Vaccinium de l'invention est un extrait préparé à partir de matériel issu d'au moins un végétal appartenant à une espèce choisie parmi les espèces *Vaccinium myrtillus, Vaccinium angustifollium, Vaccinium arboreum, Vaccinium arctostaphylos, Vaccinium caespitosum, Vaccinium corymbosum, Vaccinium hirsutum, Vaccinium macrocarpum, Vaccinium ovatum, Vaccinium oxycoccos, Vaccinium stamineum, Vaccinium uliginosum, Vaccinium urceolatum, Vaccinium vitis-idaea*.

Préférentiellement selon l'invention le végétal appartient à l'espèce *Vaccinium angustifollium.*

L'extrait d'au moins un végétal de la famille des Ericaceae peut être tout extrait préparé à partir de tout matériel végétal issu d'au moins un végétal de la famille des Ericaceae.

Ainsi, l'extrait d'au moins un végétal de la famille des Ericaceae utilisé selon l'invention peut être obtenu à partir de matériel végétal issu de plante entière ou de partie de plante comme les feuilles, les tiges, les fleurs, les pétales, les fruits, les racines ou encore des cellules dédifférenciées.

Par cellules végétales dédifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.
Selon la méthode de culture choisie, et en particulier selon le milieu de culture choisi, il est possible d'obtenir, à partir d'un même explant, des cellules végétales dédifférenciées présentant des caractères différents.

Préférentiellement selon l'invention on utilise les fruits.

L'extrait d'au moins un végétal de la famille des Ericaceae peut être tout extrait préparé à partir de tout matériel végétal issu d'au moins un végétal de la famille des Ericaceae cultivé in vivo ou issu de culture in vitro.

Par culture in vivo on entend toute culture de type classique c'est à dire en sol à l'air libre ou en serre, ou encore hors sol.

Par culture in vitro, on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales in vitro permet d'obtenir un matériel végétal standardisé et disponible tout au long de l'année contrairement aux plantes cultivées in vivo.

Préférentiellement selon l'invention on utilise un végétal issu de culture in vivo.

Toute méthode d'extraction connue de l'homme du métier peut être utilisée pour préparer l'extrait contenu dans la composition selon l'invention.
On peut en particulier citer les extraits aqueux, alcooliques ou utilisant un solvant organique.
Par solvant aqueux on entend tout solvant constitué totalement ou pour part d'eau. On peut citer ainsi l'eau elle-même, les solvants hydroalcooliques en toute proportion ou encore les solvants constitués d'eau et d'un composé comme le propylène glycol en toute proportion.

Parmi les solvants alcooliques on peut citer notamment l'éthanol.

On peut également utiliser un extrait préparé par la méthode décrite dans la demande de brevet français n° 95-02379 déposée par la demanderesse.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.
D'autre part, la première étape peut avantageusement être remplacée par une opération de congélation simple des tissus végétaux (par exemple à -20°C), suivie d'une extraction aqueuse reprenant les deuxième et troisième étapes ci-dessus décrites.

Quel que soit le mode de préparation utilisé selon l'invention des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélanger dans un solvant approprié avant utilisation.

Préférentiellement, selon l'invention on utilise un extrait aqueux et encore plus préférentiellement un extrait réalisé avec un solvant composé d'eau et de propylène glycol, comme par exemple l'Herbasol® vendu par la société COSMETOCHEM's.

Selon l'invention les extrait d'au moins un végétal de la famille des Ericaceae peuvent être utilisés seuls ou en mélanges de toute nature et peuvent être d'origine naturelle ou synthétique.

L'extrait peut constituer à lui seul le principe actif des compositions de l'invention.

Particulièrement l'extrait d'au moins un végétal de la famille des Ericaceae ou la composition le contenant sont utilisés selon l'invention en application topique sur la peau et/ou les ongles et/ou les cheveux.

La quantité d'extrait utilisable selon l'invention est bien entendu fonction de l'effet recherché et peut donc varier dans une large mesure.

Pour donner un ordre de grandeur, on peut utiliser l'extrait à l'état pur en une quantité représentant de 0,00001% à 20% du poids total de la composition et préférentiellement en une quantité représentant de 0,0001% à 10% du poids total de la composition.

La composition de l'invention peut être sous toutes formes galéniques imaginables, adaptées aussi bien à une application topique sur la peau et/ou les muqueuses et/ou les cheveux qu'à une administration par la voie orale.
De manière préférentielle, la composition de l'invention est destinée à une administration par la voie orale.

La composition de l'invention peut être une composition cosmétique ou dermatologique. Préférentiellement selon l'invention, la composition est une composition cosmétique. La composition est une composition cosmétique car elle est destinée à améliorer l'aspect cutané général de l'individu qui en fait usage.
Très préférentiellement la composition de l'invention est une composition cosmétique destinée à une administration par la voie orale.

Pour une administration par la voie orale, la composition de l'invention peut se présenter sous toutes les formes adaptées, particulièrement sous forme d'une solution buvable, d'un comprimé, d'une gélule, d'une capsule ou encore d'un aliment nutritionnel ou d'un complément nutritionnel.

Ladite composition comprend en outre au moins un excipient approprié adapté à l'administration orale.

Pour une administration par application topique sur la peau, les cheveux et/ou les muqueuses, la composition selon l'invention comprend bien évidemment un support cosmétiquement acceptable, c'est à dire un support compatible avec la peau, les muqueuses, les ongles, les cheveux et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et comme gélifiants lipophiles, on peut citer les argiles modifiées comme les bentones, les sels métalliques d'acides gras, la silice hydrophobe et les polyéthylènes.

La présente invention a en outre pour objet un procédé de traitement cosmétique de la peau destiné à stimuler la synthèse du collagène et/ou lutter contre les atteintes cutanées liées à l'âge et/ou à la ménopause et/ou lutter contre l'amincissement du derme et/ou combattre l'apparence de la peau molle et/ou ridée, caractérisé par le fait que l'on applique sur la peau, sur les cheveux, et/ou sur les muqueuses, ou que l'on ingère une composition cosmétique comprenant au moins un extrait d'au moins un végétal de la famille des Ericaceae.

Le procédé de traitement de l'invention est un procédé cosmétique destiné à améliorer l'aspect esthétique de l'individu.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits, de shampooings ou de compositions anti-solaires, sur la peau, ou sur les cheveux ou encore application de dentifrice sur les gencives et de manière préférentielle par administration par voie orale d'une solution buvable, d'un comprimé, d'une gélule ou d'une capsule ou encore d'un aliment nutritionnel ou d'un complément nutritionnel.

Les exemples et compositions suivants illustrent l'invention sans la limiter aucunement. Dans les compositions les proportions indiquées sont des pourcentages en poids.

Exemple 1 : Evaluation de l'activité d'au moins un extrait d'au moins un végétal de la famille des Ericaceae, sur les collagénases interstitielles :

L'effet d'un extrait d'au moins un végétal de la famille des Ericaceae sur la production de la collagénase interstitielle a été évalué dans un modèle de culture de cellules A2058 (issues de mélanomes humains : Templeton N.S. et al. 1990 ; Cancer Res., 50 : 5431-5431).
L'extrait testé est celui vendu par la société COSMETOCHEM's (Allemagne), commercialisé sous le nom d'Herbasol®.
Les cellules A2058 sont incubées dans un milieu DMEM contenant des acides aminés à la concentration de 2 mM, du pyruvate de sodium à la concentration de 1 mM et du sérum de veau à 10%. Elles sont ensuite mises en culture à la densité de 50000 cellules par puits dans les 24 puits de plaques multipuits.
Vingt quatre heures après la mise en culture, les cellules sont mises en contact avec un extrait d'au moins un végétal de la famille des Ericaceae. La production de la collagénase interstitielle est évaluée 48 heures plus tard dans le milieu de culture. Celle-ci est réalisée à l'aide d'un kit Elisa (Biotrack human MMP1; Amersham)
L'extrait d'au moins un végétal de la famille des Ericaceae est testé à 0,005%, 0,05% et 0,5%.

Les résultats exprimés en pourcentage représentent la diminution de la production de la collagénase interstitielle par rapport au contrôle, c'est à dire par rapport à une culture effectuée dans les mêmes conditions en l'absence d'un végétal de la famille des Ericaceae aux concentrations indiquées.

| | | | |
|---|---|---|---|
| Herbasol® | 0,05% | 0,5% | 5% |
| % d'inhibition | 0% | 7% | 53% |

Exemple 2 : Exemples de formulations illustrant l'invention et particulièrement les compositions selon l'invention. Ces compositions ont été obtenues par simple mélange des différents composants.

| Composition 1 - Capsules molles : | |
|---|---|
| Excipients : | |
| Huile de Soja | 40 mg |
| Huile de Germe de Blé | 85 mg |
| Lécithines de Soja | 25 mg |

| Vitamine : | |
|---|---|
| Tocophérols naturels | 3 mg |

| Composants : | |
|---|---|
| Herbasol® (extrait de *Vaccinium angustifollium*) | 50 mg |

| Composition 2 : Shampooing | |
|---|---|
| Herbasol® (extrait de *Vaccinium angustifollium*) | 5,00 % |
| Hydroxypropylcellulose (Klucel H® vendu par la société Hercules) | 1,00 % |
| Parfum | 0,50 % |
| Conservateur | 0,30 % |
| Eau | qsp 100,00 % |

| Composition 3 : Crème de soin du visage (émulsion huile dans eau) | |
|---|---|
| Herbasol® (extrait de *Vaccinium angustifollium*) | 2.50 % |
| Stéarate de glycérol | 2,00 % |
| Polysorbate 60 (Tween 60® vendu par la société ICI) | 1,00 % |
| Acide stéarique | 1,40 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100,00 % |

| Composition 4 : Gel pour la peau | |
|---|---|
| Herbasol® (extrait de *Vaccinium angustifollium*) | 1,00 % |
| Acide tout trans rétinoïque | 0,05 % |
| Hydroxypropylcellulose (Klucel H® vendu par la société Hercules) | 1,00 % |
| Antioxydant | 0,05 % |
| Isopropanol | 40,00 % |
| Conservateur | 0,30 % |
| Eau | qsp 100,00 % |

| Composition 5 : Gel pour le soin du visage | |
|---|---|
| Herbasol® (extrait de *Vaccinium angustifollium*) | 3,00 % |
| Hydroxypropylcellulose (Klucel H® vendu par la société Hercules) | 1,00 % |
| Antioxydant | 0,05 % |
| Isopropanol | 40,00 % |
| Conservateur | 0,30 % |
| Eau | qsp 100,00 % |

| Composition 6 : Gel | |
|---|---|
| Herbasol® (extrait de *Vaccinium angustifollium*) | 5,00 % |
| Hydroxypropylcellulose (Klucel H® vendu par la société Hercules) | 1,00 % |
| Antioxydant | 0,05 % |
| Chlorhydrate de lidocaïne | 2,00 % |
| Isopropanol | 40,00 % |
| Conservateur | 0,30 % |
| Eau | qsp 100,00 % |

| Composition 7 : Crème de soin de l'érythème solaire (émulsion huile-dans-eau) | |
|---|---|
| Herbasol® (extrait de *Vaccinium angustifollium*) | 0,50 % |
| Stéarate de glycérol | 2,00 % |
| Polysorbate 60 (Tween 60® vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00 % |
| Antioxydant | 0,05 % |
| Parfum | 0,50 % |
| Conservateur | 0,30 % |
| Eau | qsp 100,00 % |

| Composition 8 : Crème de soin antirides pour le visage (émulsion huile/eau) | |
|---|---|
| Herbasol® (extrait de *Vaccinium angustifollium*) | 1,50 % |
| Stéarate de glycérol | 2,00 % |
| Polysorbate 60 (Tween 60® vendu par la société ICI) | 1,00 % |
| Acide stéarique | 1,40 % |
| Acide n-octanoyl-5-salicylique | 0,50 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100,00 % |

| Composition 9 : Lotion | |
|---|---|
| Herbasol® (extrait de *Vaccinium angustifollium*) | 0,75 % |
| Acide glycolique | 50,00 % |
| Hydroxypropylcellulose (Klucel H® vendu par la société Hercules) | 0,05 % |
| Conservateur | 0,30 % |
| NaOH | qsp pH = 2,8 |
| Ethanol | qsp 100,00 % |

| Composition 10 : Lotion démaquillante pour le visage | |
|---|---|
| Herbasol® (extrait de *Vaccinium angustifollium*) | 0,10 % |
| Antioxydant | 0,05 % |
| Isopropanol | 40,00 % |
| Conservateur | 0,30 % |
| Eau | qsp 100,00 % |

## Revendications

1. Utilisation d'au moins un extrait d'au moins un végétal de la famille des Ericaceae, dans ou pour la préparation d'une composition, l'extrait ou la composition étant destinés à inhiber l'expression des métalloprotéinases.

2. Utilisation selon la revendication 1, **caractérisée par le fait que** l'extrait ou la composition sont destinés à inhiber l'expression de la métalloprotéinase de type 1.

3. Utilisation selon l'une quelconque des revendications précédentes, **caractérisée par le fait que** l'extrait ou la composition sont destinés à lutter contre les dégradations du collagène.

4. Utilisation selon l'une quelconque des revendications précédentes, **caractérisée par le fait que** l'extrait ou la composition sont destinés à lutter contre les dégradations internes de la peau consécutives à une exposition aux rayonnements ultra-violets.

5. Utilisation selon l'une quelconque des revendications précédentes, **caractérisée par le fait que** l'extrait ou la composition sont destinés à traiter, de manière préventive et/ou curative, les signes cutanés du vieillissement.

6. Utilisation selon l'une quelconque des revendications précédentes, **caractérisée par le fait que** l'extrait ou la composition sont destinés à traiter les atteintes cutanées liées à la ménopause.

7. Utilisation selon l'une quelconque des revendications précédentes, **caractérisée par le fait que** l'extrait ou la composition sont destinés à lutter contre les rides et ridules.

8. Utilisation selon l'une quelconque des revendications précédentes, **caractérisée par le fait que** l'extrait ou la composition sont destinés à lutter contre la peau flétrie.

9. Utilisation selon l'une quelconque des revendications précédentes, **caractérisée par le fait que** l'extrait ou la composition sont destinés à lutter contre la peau molle.

10. Utilisation selon l'une quelconque des revendications précédentes, **caractérisée par le fait que** l'extrait ou la composition sont destinés à lutter contre la peau amincie.

11. Utilisation selon l'une quelconque des revendications précédentes, **caractérisée par le fait que** l'extrait est d'origine naturelle ou synthétique.

12. Utilisation selon l'une quelconque des revendications précédentes, **caractérisée par le fait que** l'extrait est sous forme purifiée ou sous la forme d'une solution.

13. Utilisation selon l'une quelconque des revendications précédentes, **caractérisée par le fait que** l'extrait est utilisé en une quantité représentant de 0,00001% à 20% du poids total de la composition.

14. Utilisation selon l'une quelconque des revendications précédentes, **caractérisée par le fait que** l'extrait est utilisé en une quantité représentant de 0,001% à 10% du poids total de la composition.

15. Utilisation selon l'une quelconque des revendications précédentes, **caractérisée par le fait que** le végétal de la famille des Ericaceae est un végétal choisi parmi les genres Erica, Vaccinium, Calluna, Cassiope ou encore Rhododendron.

16. Utilisation selon la revendication précédente, **caractérisée par le fait que** le végétal de la famille des Ericaceae est du genre Vaccinium.

17. Utilisation selon l'une quelconque des revendications précédentes, **caractérisée par le fait que** le végétal de la famille des Ericaceae est d'une espèce choisie parmi les espèces *Vaccinium myrtillus, Vaccinium angustifollium, Vaccinium arboreum, Vaccinium arctostaphylos, Vaccinium caespitosum, Vaccinium corymbosum, Vaccinium hirsutum, Vaccinium macrocarpum, Vaccinium ovatum, Vaccinium oxycoccos, Vaccinium stamineum, Vaccinium uliginosum, Vaccinium urceolatum, Vaccinium vitis-idaea*.

18. Utilisation selon la revendication précédente, **caractérisée par le fait que** le végétal de la famille des Ericaceae est de l'espèce *Vaccinium angustifollium*.

19. Utilisation selon l'une quelconque des revendications précédentes, dans une composition destinée à une administration par la voie orale.

20. Utilisation selon la revendication précédente, **caractérisé par le fait que** la composition se présente sous la forme d'une solution buvable, d'un sirop, d'un comprimé, d'une gélule, d'une capsule ou encore d'un aliment nutritionnel ou d'un complément nutritionnel.

21. Procédé de traitement cosmétique de la peau destiné à lutter contre les atteintes cutanées liées à l'âge et/ou à la ménopause et/ou lutter contre l'amincissement du derme et/ou combattre l'apparence de la peau molle et ridée, **caractérisé par le fait que** l'on applique sur la peau, sur les cheveux, et/ou sur les muqueuses, ou que l'on ingère une composition cosmétique comprenant au moins d'au moins un extrait d'au moins un végétal de la famille des Ericaceae.
